# EUROPÄISCHE PATENTSCHRIFT

(11) **EP 2 735 267 B1**
(45) Veröffentlichungstag und Bekanntmachung des Hinweises auf die Patenterteilung: **04.01.2023**
(21) Anmeldenummer: 13189841.3
(22) Anmeldetag: 23.10.2013
(51) Int. Cl.: A61B 5/03, A61M 5/168, A61B 5/01

(54) **SET AUS EINER MEDIZINISCHEN DRUCKSENSOREINRICHTUNG UND MINDESTENS EINEM WECHSEL-KATHETER**
SET COMPRISING A MEDICAL PRESSURE SENSOR DEVICE AND AT LEAST ONE EXCHANGE CATHETER
JEU CONSTITUÉ D'UN DISPOSITIF MÉDICAL DE DÉTECTION DE PRESSION ET AU MOINS UN CATHÉTER DE RECHANGE

(30) Priorität: 21.11.2012 DE 102012221284
(43) Veröffentlichungstag der Anmeldung: 28.05.2014
(73) Patentinhaber: RAUMEDIC AG, 95213 Münchberg (DE)
(72) Erfinder: Göhler, Karlheinz, 08297 Zwönitz (DE); Kunze, Hans Gerd, 08297 Zwönitz (DE)
(74) Vertreter: Rau, Schneck & Hübner Patentanwälte Rechtsanwälte PartGmbB

(56) Entgegenhaltungen:
- DE-U1-202007 002 592
- US-A- 4 206 762
- US-A1- 2004 215 162
- US-A1- 2010 094 143
- US-A1- 2012 232 462

## Beschreibung

Die Erfindung betrifft ein Set aus einer medizinischen Drucksensoreinrichtung mit mindestens einem Wechsel-Katheter zur Messung eines Drucks eines Körperfluids mit einem Basisgehäuse und einem hiermit verbundenen Katheter.

Eine medizinische Drucksensoreinrichtung ist bekannt aus der EP 1 521 723 B1, der WO 2009/106284 A1, der US 4,127,110, der US 4,519,401, der US 4,281,667, der EP 1 887 930 B1, der EP 2 022 396 A2, der EP 2 025 286 B1, der DE 20 2007 002 592 U1, der US 2004/0215162 A1, der US 2010/0094143 A1 und aus der US 2012/0232462 A1.

Es ist eine Aufgabe der vorliegenden Erfindung, ein Set mit einer Drucksensoreinrichtung der eingangs genannten Art derart weiterzubilden, dass die Anforderungen an eine Ausgestaltung enthaltener elektronischer Komponenten, also der Drucksensoreinheit und den elektronischen Bauelementen im Basisgehäuse, reduziert sind, ohne hierdurch Abstriche bei der Wahl eines gegebenenfalls vom Basisgehäuse entfernten Druck-Messorts zu machen.

Diese Aufgabe ist erfindungsgemäß gelöst durch ein Set aus einer medizinischen Drucksensoreinrichtung mit mindestens einem Wechsel-Katheter mit den im Anspruch 1 angegebenen Merkmalen.

Ein Set aus einer medizinischen Drucksensoreinrichtung kann mit Kathetern unterschiedlicher Längen und/oder unterschiedlicher Durchmesser und/oder unterschiedlicher Festigkeiten und/oder unterschiedlichen Anordnungen und Weiten der Katheter-Durchgangsöffnungen ausgerüstet sein.

Zu dem Set können Katheter aus verschiedenen Materialien gehören. Auch Katheter aus verschiedenen Materialkombinationen zur Vorgabe von über die Länge des Katheters unterschiedlichen Festigkeiten bzw. Steifigkeiten sind möglich. Erfindungsgemäß wurde weiter erkannt, dass eine Anordnung der Drucksensoreinrichtung im Basisgehäuse die Anforderungen an eine Signalverbindung zwischen der Drucksensoreinheit und den elektronischen Bauelementen zur Steuerung und zum Auslesen der Drucksensoreinheit im Basisgehäuse entscheidend reduziert. Auslesen bedeutet, dass von der Drucksensoreinheit ermittelte Messwerte an die elektronischen Bauelemente übertragen werden. Ein Speicherelement ist somit nicht zwingend erforderlich, kann aber in der Drucksensoreinheit integriert oder zumindest mit dieser verbunden sein. Störungen aufgrund von Übertragungswegen zwischen der Drucksensoreinheit und den elektronischen Bauelementen können daher minimiert werden. Auch der Fertigungsaufwand der Drucksensoreinrichtung ist reduziert, da sämtliche, miteinander in Signalverbindung stehenden Komponenten der Drucksensoreinrichtung gemeinsam im Basisgehäuse montiert werden können. Die Bauelemente zur Steuerung und zum Auslesen der Drucksensoreinheit sind auf einer Leiterplatte im Basisgehäuse angeordnet. Die Drucksensoreinheit ist ebenfalls auf dieser Leiterplatte angeordnet. Bei der Drucksensoreinrichtung handelt es sich um einen Hirndrucksensor. Die Drucksensoreinrichtung kann mehrere Drucksensoreinheiten und/oder mehrere zusätzliche Sensoren, zum Beispiel mindestens einen Temperatursensor, aufweisen. Die Drucksensoreinrichtung kann zur telemetrischen Verbindung mit einem externen Gerät ausgeführt sein. Der Druck-Messort wird durch die Position der Katheter-Durchgangsöffnung vorgegeben. Je nach erwünschtem Druck-Messort kann daher ein Katheter mit einer entsprechend angepassten Form sowie einer entsprechend angepassten Größe und Anordnung der Katheter-Durchgangsöffnung gewählt werden. Gemessen werden kann sowohl ein physiologischer als auch ein pathologischer Hirndruck eines Patienten.

Verbindungsvarianten nach den Ansprüchen 2 bis 6 haben sich als ausreichend robust herausgestellt. Es kann eine fluiddichte Verbindung zwischen dem Katheter und dem Basisgehäuse hergestellt werden.

Eine Abdichtung nach Anspruch 8 verringert die Anforderungen an die Bauelemente zur Steuerung und zum Auslesen der Drucksensoreinheit.

Eine Sensorausführung mit einer drucksensitiven Fläche in Form einer Membran nach Anspruch 9 hat sich in der Praxis bewährt. Die drucksensitive Fläche kann aus Silizium gefertigt sein.

Eine nichtorganische Beschichtung nach Anspruch 10 kann die drucksensitive Fläche gegen eine unerwünschte Beeinflussung durch das über die Fluidverbindung eindringende Körperfluid schützen. Die nichtorganische Beschichtung kann in einem halbleitertechnologischen Prozess aufgebracht werden und beispielsweise auch gesputtert werden. Die nichtorganische Beschichtung kann die einzige Beschichtung auf der drucksensitiven Fläche sein.

Eine Silikon-Beschichtung nach Anspruch 11 kann alternativ oder zusätzlich auf der drucksensitiven Fläche aufgebracht sein. Die Silikon-Beschichtung kann dünn ausgeführt sein und beispielsweise eine Schichtstärke im Bereich zwischen 100µm und 0,5mm haben. Die Silikon-Beschichtung kann die drucksensitive Fläche gegen eine unerwünschte Beeinflussung durch das über die Fluidverbindung eindringende Körperfluid schützen.

Ausführungsbeispiele der Erfindung werden nachfolgend anhand der Zeichnung näher erläutert. In dieser zeigen:
- Fig. 1: einen in Bezug auf einen Katheter abgebrochenen Längsschnitt durch eine medizinische Drucksensoreinrichtung zur Messung eines physiologischen Drucks;

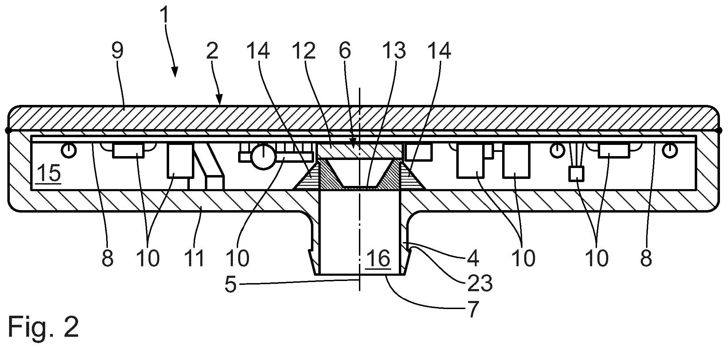

- Fig. 2: in einer zu Fig. 1 ähnlichen Darstellung einen Querschnitt durch ein Basisgehäuse der Drucksensoreinrichtung, enthaltend eine Drucksensoreinheit sowie elektronische Bauelemente zur Steuerung und zum Auslesen von dieser; und
- Fig. 3 bis 6: jeweils in einer Seitenansicht Ausführungen von Kathetern, die mit dem Basisgehäuse über einen Verbindungsstutzen wahlweise verbindbar sind.

Eine medizinische Drucksensoreinrichtung 1 dient zur Messung eines physiologischen Drucks, beispielsweise eines Hirndrucks.

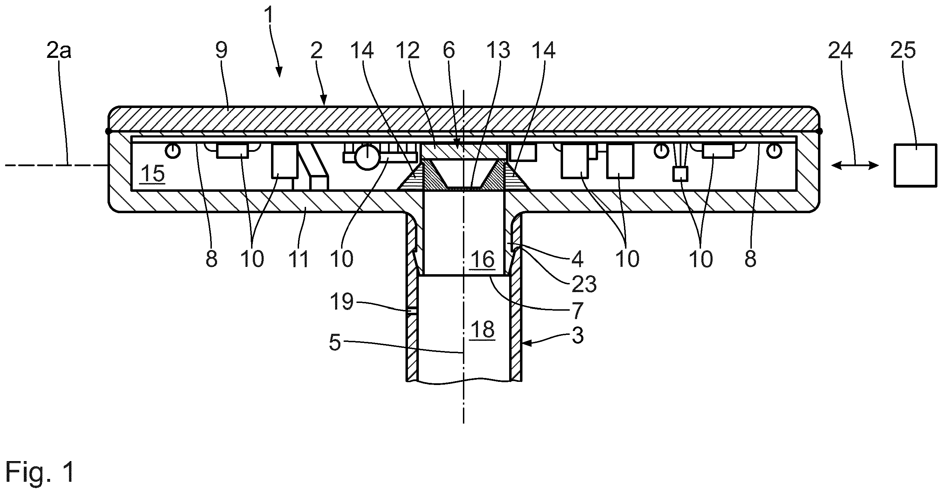

Die Drucksensoreinrichtung 1 hat ein Basisgehäuse 2 mit einer scheibenförmigen Grundform, wobei eine Scheibenebene 2a senkrecht zur Zeichenebene der Fig. 1 verläuft. Das Basisgehäuse 2 ist insbesondere zum Aufsetzen auf eine Schädelkalotte eines Patienten ausgebildet. Weiterhin hat die Drucksensoreinrichtung 1 einen in der Fig. 1 lediglich abschnittsweise dargestellten Katheter 3, der mit dem Basisgehäuse 2 verbunden ist. Der Katheter erstreckt sich senkrecht zur Scheibenebene 2a. Der Katheter 3 ist aus Kunststoff. Ein Grundkörper des Katheters 3 ist als Katheterschlauch ausgeführt. Der grundsätzliche Aufbau einer Drucksensoreinrichtung ist bekannt aus der EP 1 531 723 B1 und der WO 2009/106284 A1.

Der Katheterschlauch ist aus einem Silikonmaterial. Grundsätzlich kann der Katheterschlauch aus Silikonen, Polyurethanen, Polyamiden, Fluorpolymeren, Polyolefinen und Copolymeren dieser Materialien ausgeführt sein.

Ein Innenlumen des Katheters 3 kann ein sehr kleines Volumen aufweisen. Dieses Volumen liegt im Bereich <50µl und kann kleiner sein als 30µl und beispielsweise 25µl betragen. Auch ein noch kleineres Volumen des Innenlumens des Katheters 3 kann je nach Ausführung vorhanden sein.

Zu Verbindung des Katheters 3 mit dem Basisgehäuse 2 hat das Basisgehäuse 2 einen Verbindungsstutzen 4, der nach Art eines Anschlussstutzens eines Fittings ausgeführt ist und auch als Schlauchtülle oder als Olivenstutzen bezeichnet wird.

Der Verbindungsstutzen 4 erstreckt sich längs einer Stutzen-Längsachse 5, die mit einer Rotationssymetrieachse der Scheiben-Grundform des Basisgehäuses 2 zusammenfällt.

Das Basisgehäuse 2 beinhaltet eine Drucksensoreinheit 6. Diese ist gegenüber einer Öffnung 7 des Verbindungsstutzens 4 des Basisgehäuses 2 angeordnet, ist also zentral auf der Stutzen-Längsachse 5 im Basisgehäuse 2 angeordnet. Um die Drucksensoreinheit 6 herum sind im Basisgehäuse 2 auf einer Leiterplatte 8 elektronische Bauelemente 10 zur Steuerung und zum Auslesen der Drucksensoreinheit 6 angeordnet.

Das Basisgehäuse 2 umfasst ein Gehäuseoberteil 9, das das Basisgehäuse 2 nach oben hin, bei der Verwendung als Hirnparametersensor also zur Kopfhaut des Patienten hin, abdeckt und ein hiermit verbundenes Gehäuseunterteil 11. Das Gehäuseunterteil 11 ist mit dem Gehäuseoberteil 9 umlaufend um die Symmetrieachse 5 verklebt. Der Verbindungsstutzen 4 ist einstückig in das Gehäuseunterteil 11 eingeformt.

Das Gehäuseoberteil 9 und das Gehäuseunterteil 11 sind aus einer biokompatiblen Keramik. Alternativ ist auch eine Ausführung aus biokompatiblem Kunststoff, z. B. Polyurethan, Polyamid, Fluorpolymer, Silikon oder mindestens einem Copolymer aus diesen Materialien, möglich.

Die Drucksensoreinheit 6 umfasst einen Absolutdrucksensor bzw. ein Druckmessmodul 12, der auf der Leiterplatte 8 aufgebracht ist. Weiterhin umfasst die Drucksensoreinheit 6 eine drucksensitive Fläche 13. Diese ist bei der dargestellten Ausführung als drucksensitive Membran ausgeführt. Die drucksensitive Fläche 13 ist zwischen dem Absolutdrucksensor 12 und der Öffnung 7 des Verbindungsstutzens 4 angeordnet.

Die drucksensitive Fläche 13 kann eine Silikon-Beschichtung aufweisen. Die drucksensitive Fläche 13 selbst ist aus Silizium ausgeführt. Alternativ oder zusätzlich zur Silikon-Beschichtung kann auf der Oberfläche der drucksensitiven Fläche 13 eine Schicht aus thermischem Oxid, insbesondere aus SiO₂ ausgeführt sein. Eine Beschichtung der drucksensitiven Fläche 13, die diese gegen äußere Einflüsse schützen kann, kann auch aus nichtorganischen Materialien ausgeführt sein. Diese Schutzschicht kann mittels halbleitertechnologischer Prozesse, z. B. durch Sputtern, aufgebracht werden. Eine solche Schutzschicht kann als Metallschicht ausgeführt sein.

Mittels einer um die Stutzen-Längsachse 5 umlaufenden Ringdichtung 14 ist die Drucksensoreinheit 6 gegen das Gehäuseunterteil 11 abgedichtet. Die Ringdichtung 14 liegt dabei einerseits umlaufend an der drucksensitiven Fläche 13 und andererseits an einer Innenwand des Gehäuseunterteils 11 im Übergangsbereich zum Verbindungsstutzen 4 an. Durch die Ringdichtung 14 ist gewährleistet, dass die Bauelemente 10 in mindestens einem Raum, nämlich im Innenraum 15 des Basisgehäuses 2 angeordnet sind, der gegenüber einer Fluidverbindung 16, die durch den Verbindungsstutzen 4 geschaffen ist, abgedichtet ist. Die Silikon-Beschichtung der drucksensitiven Fläche 13 kann zumindest auf der Seite der drucksensitiven Fläche 13 ausgeführt sein, die der Fluidverbindung 16 zugewandt ist.

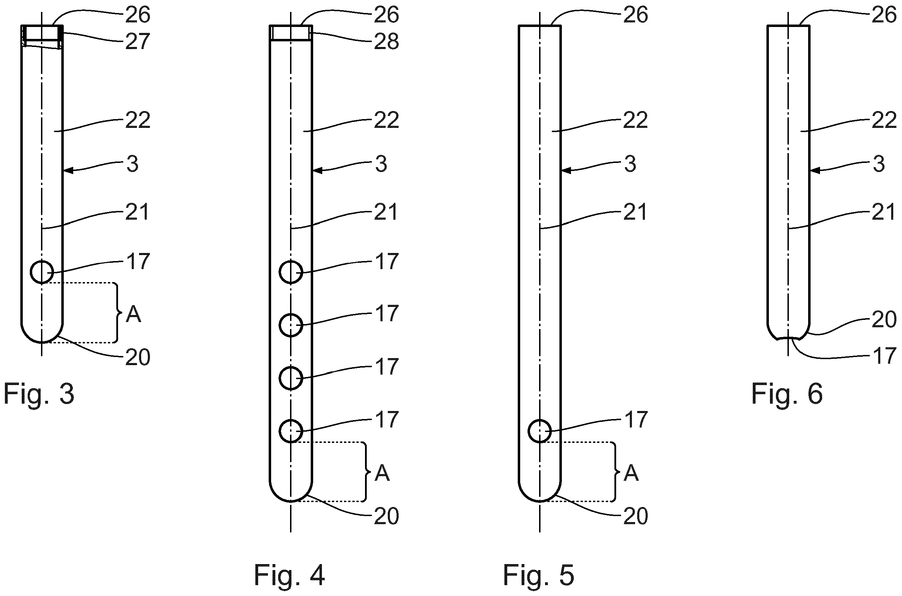

Der Katheter 3 hat je nach Ausführung (vgl. die Fig. 3 bis 6) mindestens eine Katheter-Durchgangsöffnung 17. Ein Innenlumen 18 des Katheters 3 und der Verbindungsstutzen 4 bilden die Fluidverbindung 16, über die ein Fluid, das den zu messenden Druck vermittelt, die Drucksensoreinheit 6 von der jeweiligen Katheter-Durchgangsöffnung 17 her erreichen kann. Die Katheter-Durchgangsöffnung 17 gibt einen von der Drucksensoreinrichtung 6 entfernten Druck-Messort vor.

Nahe der Öffnung 7 des Verbindungsstutzens 4 kann der Katheter 3 mindestens eine Entlüftungsöffnung 19 aufweisen. Varianten der Katheter 3, die in der Zeichnung nicht dargestellt sind, können auch ohne eine derartige Entlüftungsöffnung ausgeführt sein.

Bei der Katheter-Ausführung nach Fig. 3 liegt genau eine Katheter-Durchgangsöffnung 17 vor einem freien Ende 20 des Katheters 3 vor. Die Katheter-Durchgangsöffnung 17 ist dabei nahe dem freien Ende 20 angeordnet. Ein Abstand A zwischen der Katheter-Durchgangsöffnung 17 und dem freien Ende 20 beträgt dabei etwa ein Fünftel der Gesamtlänge des Katheters 3.

Bei der Ausführung nach Fig. 4 weist der Katheter 3 insgesamt 4 Katheter-Durchgangsöffnungen 17 auf, die längs einer Katheter-Längsachse 21, die bei montiertem Katheter 3 mit der Stutzen-Längsachse 5 zusammenfällt, aufgereiht angeordnet sind. Bei der Ausführung nach Fig. 4 haben die vier Katheter-Durchgangsöffnungen 17 gleichen Abstand zueinander und sind jeweils an der gleichen Umfangsposition relativ zur Katheter-Längsachse 21 in einer Mantelwand 22 des Katheters 3 ausgeführt. Alternativ ist es möglich, die Katheter-Durchgangsöffnungen 17 verschieden zueinander zu beabstanden und/oder auch an verschiedenen Umfangspositionen um die Katheter-Längsachse 21 anzuordnen. Ein Abstand A der bei montiertem Katheter 3 zum Verbindungsstutzen 4 entferntesten Katheter-Durchgangsöffnung 17 zum freien Ende 20 des Katheters entspricht dem Abstand der Katheter-Durchgangsöffnung 17 zum freien Ende 20 bei der Ausführung nach Fig. 3.

Die dem Verbindungsstutzen 4 nächst benachbarte Durchgangsöffnung 17 ist bei der Ausführung nach Fig. 4 etwa auf der halben Länge des Katheters 3 ausgeführt.

Der Katheter 3 ist bei der Ausführung nach Fig. 4 etwa eineinhalb mal so lang wie bei der Ausführung nach Fig. 3.

Die Ausführung des Katheters 3 nach Fig. 5 ist etwa so lang wie die Ausführung nach Fig. 4, hat aber nur genau eine Katheter-Durchgangsöffnung 17, die wiederum im Abstand A zum freien Ende 20 des Katheters 3 nach Fig. 5 angeordnet ist.

Bei der Ausführung nach Fig. 6 ist die Katheter-Durchgangsöffnung 17 zentral im freien Ende 20 des Katheters 3 ausgeführt. Die Durchgangsöffnung 17 liegt also rotationssymmetrisch um die Katheter-Längsachse 21. Bei montiertem Katheter 3 nach Fig. 6 fluchtet die Katheter-Durchgangsöffnung 17 mit dem Verbindungsstutzen 4 mit der Drucksensoreinheit 6.

Bei einer weiteren, nicht dargestellten Variante des Katheters 3 ist die Ausführung nach Fig. 6 mit endseitig zentral angebrachter Katheter-Durchgangsöffnung 17 mit weiteren Katheter-Durchgangsöffnungen 17 kombiniert, die nach Art derjenigen ausgeführt und angeordnet sind, die vorstehend im Zusammenhang mit den Katheter-Ausführungen nach den Fig. 3 bis 5 bereits erläutert wurden.

Die Katheter-Durchgangsöffnungen 17 sind rund ausgeführt. Alternativ ist es möglich, die Katheter-Durchgangsöffnungen mit anderer Öffnungsform, zum Beispiel quadratisch, rechteckig oder schlitzförmig auszuführen. Soweit mehrere Katheter-Durchgangsöffnungen 17 in einer Ausführung des Katheters 3 ausgeführt sind, können diese genau gleich groß oder auch unterschiedlich groß ausgeführt sein.

Die verschiedenen Katheter 3 des Sets können sich in ihrer Länge und/oder in ihrem Durchmesser und/oder in ihrer Materialfestigkeit und/oder in der Anordnung bzw. Anzahl der Katheter-Durchgangsöffnungen 17 unterscheiden.

Die medizinische Drucksensoreinrichtung 1 bildet zusammen mit mehreren Kathetern 3 ein Set. Dabei kann einer der Katheter 3 bereits mit dem Basisgehäuse 2 über den Verbindungsstutzen 4 verbunden sein. Der mindestens eine weitere Katheter 3 des Sets stellt dann einen Wechsel-Katheter dar, der im Austausch mit dem bereits verbundenen Katheter 3 mit dem Basisgehäuse 2 verbindbar ist. Alternativ können bei dem Set zunächst alle Katheter 3 nicht mit dem Basisgehäuse 2 verbunden sein. Die verschiedenen Katheter 3 des Sets können unterschiedlich angeordnete Durchgangsöffnungen 17 und/oder können unterschiedlich große Katheter-Durchgangsöffnungen 17 aufweisen.

Die medizinische Drucksensoreinrichtung 1 kommt folgendermaßen zum Einsatz: Abhängig von dem zu messenden Druck und abhängig vom Einsatzort der Drucksensoreinrichtung 1 wird zunächst eine Fixierung des Basisgehäuses 2 vorbereitet und es wird ein zum Einsatzort passender Katheter 3 ausgewählt. Diese Vorbereitung und Auswahl erfolgt zudem abhängig vom Patienten. Ein Kind benötigt beispielsweise einen dünneren und kürzeren Katheter 3 als ein Erwachsener. Anschließend wird der ausgewählte Katheter 3 durch Aufschieben eines gehäuseseitigen Katheterendes auf dem Verbindungsstutzen 4 mit dem Basisgehäuse 2 verbunden. Diese Verbindung ist einerseits fluiddicht und andererseits so fest, dass ein unerwünschtes Lösen des Katheters 3 vom Verbindungsstutzen 4 verhindert ist. Hierzu weist der Verbindungsstutzen 4 eine umlaufende Hinterschneidung 23 auf, die sich in eine Innenwand des Katheters 3 einarbeitet und so eine das Lösen verhindernde Verzahnung mit dieser Innenwand bildet. Nun wird die montierte Drucksensoreinrichtung 1 an ihren Einsatzort verbracht, an dem der Katheter 3 mit seiner mindestens einen Katheter-Durchgangsöffnung 17 zum Messort des Körperfluids, dessen Druck gemessen werden soll, gelangt. Beispielsweise kann der Katheter mit der Katheter-Öffnung im Parenchym oder im Bereich der Ventrikel angeordnet sein.

Das Messfluid gelangt nun über die mindestens eine Katheter-Druchgangsöffnung 17 des Katheters 3 durch den Katheter hin zur drucksensitiven Fläche 13 der Drucksensoreinheit 6. Gegebenenfalls durch das Messfluid verdrängte Luft oder Gas kann aus dem Katheter 3 durch die Entlüftungsöffnung 19 entweichen.

Der Druck, den das Messfluid auf die drucksensitive Fläche 13 ausübt, stellt ein Maß für den zu messenden physiologischen Druck dar. Dieser wird von der drucksensitiven Fläche 13 auf den Absolutdrucksensor 12 übertragen.

Der gemessene Druck wird mithilfe der Bauelemente 10 digitalisiert, ausgelesen und über eine Telemetrie-Verbindung 24, die in der Fig. 1 schematisch durch einen Doppelpfeil angedeutet ist, an ein externes Gerät 25, beispielsweise einen Auswerterechner, weitergeleitet. Über die Telemetrie-Verbindung 24 ist eine drahtlose Übertragung der Druckdaten möglich.

Über die Bauelemente 10 ist zudem eine Steuerung der Drucksensoreinheit 6 möglich, beispielsweise eine Vorwahl einer Druckmessempfindlichkeit. Die Steuerdaten können der Drucksensoreinheit 6 auch über die Telemetrie-Verbindung 24 von extern übermittelt werden.

Über die Telemetrie-Verbindung 24 ist auch insgesamt eine Aktivierung und auch eine Deaktivierung der Drucksensoreinrichtung 1 insgesamt möglich.

Zu den elektronischen Bauelementen 10 gehören eine Sende-/Empfangsantenne für die Telemetrie-Verbindung 24 und eine Batterie. Zu den elektronischen Bauelementen 10 gehört auch ein Datenspeicher für die Druckdaten. Je nach Ausführung der Drucksensoreinrichtung 1 kann auf eine Batterie und/oder kann auf einen Datenspeicher für die Druckdaten auch verzichtet werden.

Die Datenverbindung zwischen der Drucksensoreinrichtung 1 und dem externen Gerät 25 kann auch über RFID (Radio Frequency Identification; Identifizierung mithilfe elektromagnetischer Wellen) erfolgen.

Das externe Gerät 25 kann als Datalogger oder auch als Monitoreinrichtung ausgeführt sein. Das externe Gerät 25 kann beispielsweise Teil eines Patientenzimmer-Equipments in einem Krankenhaus sein.

Alternativ zu einer Verbindung des Katheters 3 mit dem Basisgehäuse 2 über einen Verbindungsstutzen ist eine Verbindung auch über eine Schraubverbindung möglich. Hierzu kann ein Verbindungsende 26 des jeweiligen Katheters 3, das dem freien Ende 20 gegenüberliegend angeordnet ist, ein Innengewinde aufweisen, das in der Fig. 3 stellvertretend für die verschiedenen Ausführungen des Katheters 3 bei 27 angedeutet ist. Dieses Innengewinde 27 ist komplementär zu einem Außengewinde in einer Außenwand eines alternativ ausgeführten Verbindungsstutzens 4 ausgeführt.

Bei einer weiteren Ausführung kann das Gehäuseunterteil 10 im Bereich der Fluidverbindung 16 ein Innengewinde aufweisen, welches wiederum komplementär zu einem Außengewinde 28 (vergleiche stellvertretend die Ausführung nach Fig. 4) am Verbindungsende 26 des Katheters 3 ausgeführt ist.

## Patentansprüche

1. Set aus einer medizinischen Drucksensoreinrichtung (1) zur Messung eines Hirndrucks
- mit einer Drucksensoreinheit (6),
- mit einem Basisgehäuse (2), enthaltend elektronische Bauelemente (10) zur Steuerung und zum Auslesen der Drucksensoreinheit (6), wobei das Basisgehäuse (2) ein Gehäuseoberteil (9) und ein hiermit verbundenes Gehäuseunterteil (11) umfasst,
- mit genau einem Katheter (3), der über einen Verbindungsstutzen (4) mit dem Basisgehäuse (2) verbindbar ist,
- wobei der Katheter (3) mindestens eine Katheter-Durchgangsöffnung (17) aufweist, die mit der Drucksensoreinheit (6) in Fluidverbindung (16) steht,
- wobei die Drucksensoreinheit (6) im Basisgehäuse (2) angeordnet ist,
- wobei die mindestens eine Katheter-Durchgangsöffnung (17) einen von der Drucksensoreinheit (6) entfernten Druck-Messort vorgibt,
**dadurch gekennzeichnet, dass**
- das Set ferner mindestens einen Wechsel-Katheter aufweist, der im Austausch mit dem Katheter (3) über den Verbindungsstutzen (4) mit dem Basisgehäuse (2) verbindbar ist, wobei der Katheter (3) und der mindestens eine Wechsel-Katheter unterschiedlich angeordnete Durchgangsöffnungen (17) und/oder unterschiedlich große Durchgangsöffnungen (17) aufweisen,
- ein Grundkörper des Katheters (3) als Katheterschlauch aus Kunststoff ausgeführt ist,
- die Drucksensoreinheit (6) auf einer Leiterplatte (8) im Basisgehäuse (2) zentral zu einer Stutzen-Längsachse (5) des Verbindungsstutzens (4) angeordnet ist,
- die Drucksensoreinheit (6) gegen das Gehäuseunterteil (11) mittels einer um die Stutzen-Längsachse (5) umlaufenden Ringdichtung (14) abgedichtet ist,
- die elektronischen Bauelemente (10) zur Steuerung und zum Auslesen der Drucksensoreinheit (6) um die Drucksensoreinheit (6) herum auf der Leiterplatte (8) in einem Innenraum (15) des Basisgehäuses (2) angeordnet sind,
- der Innenraum (15) gegenüber einer durch den Verbindungsstutzen (4) geschaffenen Fluidverbindung (16) abgedichtet ist.

2. Set nach Anspruch 1, **dadurch gekennzeichnet, dass** der Verbindungsstutzen (4) Teil des Basisgehäuses (2) ist.

3. Set nach Anspruch 1 oder 2, **dadurch gekennzeichnet, dass** der Verbindungsstutzen (4) als Anschlussstutzen eines Fittings ausgeführt ist.

4. Set nach Anspruch 3, **dadurch gekennzeichnet, dass** der Verbindungsstutzen (4) eine umlaufende Hinterschneidung (23) aufweist, die sich in eine Innenwand des Katheters (3) einarbeitet und so eine Verzahnung mit dieser Innenwand bildet.

5. Set nach Anspruch 3, **dadurch gekennzeichnet, dass** der Katheter (3) über eine Schraubverbindung mit dem Basisgehäuse (2) verbindbar ist.

6. Set nach Anspruch 5, **dadurch gekennzeichnet, dass** der Verbindungsstutzen (4) ein Innen- oder Außengewinde zu einem an einem Verbindungsende (26) des Katheters (3) angeordneten Außen (28) oder Innengewinde (27) aufweist.

7. Set nach einem der Ansprüche 1 bis 6, **dadurch gekennzeichnet, dass** die Drucksensoreinheit (6) gegenüber einer Öffnung (7) des Verbindungsstutzens (4) im Basisgehäuse (2) angeordnet ist.

8. Set nach einem der Ansprüche 1 bis 7, **dadurch gekennzeichnet, dass** die elektronischen Bauelemente (10) in mindestens einem Raum (15) im Basisgehäuse (2) angeordnet sind, der gegenüber der Fluidverbindung (16) zwischen der Katheter-Durchgangsöffnung (17) und der Drucksensoreinheit (6) abgedichtet ist.

9. Set nach einem der Ansprüche 1 bis 8, **dadurch gekennzeichnet, dass** die Drucksensoreinheit (6) eine als Membran ausgeführte drucksensitive Fläche (13) aufweist, die mit der Katheter-Durchgangsöffnung (17) in direkter Fluidverbindung (16) steht.

10. Set nach Anspruch 9, **dadurch gekennzeichnet, dass** die drucksensitive Fläche (13) eine nichtorganische Beschichtung trägt.

11. Set nach Anspruch 9 oder 10, **dadurch gekennzeichnet, dass** die drucksensitive Fläche (13) eine Silikon-Beschichtung trägt.

12. Set nach einem der vorstehenden Ansprüche, **dadurch gekennzeichnet, dass** das Basisgehäuse (2) aus einer biokompatiblen Keramik oder aus biokompatiblem Kunststoff, insbesondere Polyurethan, Polyamid, Fluorpolymer, Silikon oder mindestens einem Copolymer aus diesen Materialien, ausgeführt ist.

13. Set gemäß einem der vorstehenden Ansprüche, **dadurch gekennzeichnet, dass** der Katheterschlauch aus Silikonmaterial, aus Silikonen, aus Polyurethanen, aus Polyamiden, aus Fluorpolymeren, aus Polyolefinen oder aus Copolymeren dieser Materialien ausgeführt ist.

14. Set gemäß einem der vorstehenden Ansprüche, **dadurch gekennzeichnet, dass** die Ringdichtung (14) umlaufend an einer drucksensitiven Fläche (13) der Drucksensoreinheit (6) und an einer Innenwand des Gehäuseunterteils (11) im Übergangsbereich zu einem Verbindungsstutzen (4) anliegt.

## Claims

1. Set comprising a medical pressure sensor device (1) for measuring a cerebral pressure
- having a pressure sensor unit (6),
- having a base housing (2) containing electronic components (10) for controlling and reading out the pressure sensor unit (6), wherein the base housing (2) comprises a housing upper part (9) and a housing lower part (11) connected thereto,
- having exactly one catheter (3) which can be connected to the base housing (2) via a connecting piece (4),
- wherein the catheter (3) has at least one catheter passage opening (17) which is in fluid connection (16) with the pressure sensor unit (6),
- wherein the pressure sensor unit (6) is arranged in the base housing (2),
- wherein the at least one catheter passage opening (17) defines a pressure measurement location remote from the pressure sensor unit (6),
**characterized in that**
- the set further comprises at least one exchange catheter which can be connected to the base housing (2) via the connecting piece (4) in exchange with the catheter (3), wherein the catheter (3) and the at least one exchange catheter comprise differently arranged passage openings (17) and/or differently sized passage openings (17),
- a main body of the catheter (3) is designed as a catheter tube made of plastic,
- the pressure sensor unit (6) is arranged on a printed circuit board (8) in the base housing (2) centrally to a connecting piece longitudinal axis (5) of the connecting piece (4),
- the pressure sensor unit (6) is sealed against the housing lower part (11) by means of an annular seal (14) running around the connecting piece longitudinal axis (5),
- the electronic components (10) for controlling and reading out the pressure sensor unit (6) are arranged around the pressure sensor unit (6) on the printed circuit board (8) in an interior space (15) of the base housing (2),
- the interior space (15) is sealed from a fluid connection (16) created by the connecting piece (4).

2. Set according to claim 1, **characterized in that** the connection piece (4) is part of the base housing (2).

3. Set according to claim 1 or 2, **characterized in that** the connecting piece (4) is designed as a connection nozzle of a fitting.

4. Set according to claim 3, **characterized in that** the connecting piece (4) has a circumferential undercut (23) which works its way into an inner wall of the catheter (3) and thus forms an interlock with this inner wall.

5. Set according to claim 3, **characterized in that** the catheter (3) can be connected to the base housing (2) via a screw connection.

6. Set according to claim 5, **characterized in that** the connecting piece (4) has an internal or external thread to an external (28) or internal thread (27) arranged at a connection end (26) of the catheter (3).

7. Set according to any one of claims 1 to 6, **characterized in that** the pressure sensor unit (6) is arranged opposite an opening (7) of the connecting piece (4) in the base housing (2).

8. Set according to any one of claims 1 to 7, **characterized in that** the electronic components (10) are arranged in at least one space (15) in the base housing (2) which is sealed off from the fluid connection (16) between the catheter passage opening (17) and the pressure sensor unit (6).

9. Set according to any one of claims 1 to 8, **characterized in that** the pressure sensor unit (6) has a pressure-sensitive surface (13) in the form of a membrane which is in direct fluid connection (16) with the catheter passage opening (17).

10. Set according to claim 9, **characterized in that** the pressure-sensitive surface (13) carries a non-organic coating.

11. Set according to claim 9 or 10, **characterized in that** the pressure-sensitive surface (13) carries a silicone coating.

12. Set according to any one of the preceding claims, **characterized in that** the base housing (2) is made of a biocompatible ceramic or of biocompatible plastic, in particular polyurethane, polyamide, fluoropolymer, silicone or at least one copolymer of these materials.

13. Set according to any one of the preceding claims, **characterized in that** the catheter tube is made of silicone material, of silicones, of polyurethanes, of polyamides, of fluoropolymers, of polyolefins or of copolymers of these materials.

14. Set according to any one of the preceding claims, **characterized in that** the ring seal (14) bears circumferentially against a pressure-sensitive surface (13) of the pressure sensor unit (6) and against an inner wall of the housing lower part (11) in the transition region to a connecting piece (4).

## Revendications

1. Ensemble constitué d'un dispositif capteur de pression médical (1) pour la mesure d'une pression cérébrale
- ayant une unité de capteur de pression (6),
- ayant un boîtier de base (2), contenant des composants électroniques (10) pour la commande et la lecture de l'unité de capteur de pression (6), le boîtier de base (2) comprenant une partie supérieure de boîtier (9) et une partie inférieure de boîtier (11) reliée à celle-ci,
- ayant exactement un cathéter (3) qui peut être relié au boîtier de base (2) par une tubulure de liaison (4),
- dans lequel le cathéter (3) présente au moins un orifice de passage de cathéter (17) qui est en connexion fluidique (16) avec l'unité de capteur de pression (6),
- dans lequel l'unité de capteur de pression (6) est disposée dans le boîtier de base (2),
- dans lequel ledit au moins un orifice de passage de cathéter (17) définit un emplacement de mesure de pression éloigné de l'unité de capteur de pression (6),
**caractérisé en ce que**
- l'ensemble présente en outre au moins un cathéter de rechange qui peut être relié au boîtier de base (2) en échange avec le cathéter (3) par l'intermédiaire de la tubulure de liaison (4), le cathéter (3) et ledit au moins un cathéter de rechange présentant des orifices de passage (17) disposés différemment et/ou des orifices de passage (17) de tailles différentes,
- un corps principal du cathéter (3) est réalisé sous forme de tuyau de cathéter en matière plastique,
- l'unité de capteur de pression (6) est disposée sur une carte de circuit imprimé (8) dans le boîtier de base (2) de manière centrale par rapport à un axe longitudinal de tubulure (5) de la tubulure de liaison (4),
- l'unité de capteur de pression (6) est rendue étanche par rapport à la partie inférieure de boîtier (11) au moyen d'un joint annulaire (14) s'étendant autour de l'axe longitudinal de tubulure (5),
- les composants électroniques (10) pour la commande et la lecture de l'unité de capteur de pression (6) sont disposés autour de l'unité de capteur de pression (6) sur la carte de circuit imprimé (8) dans un espace intérieur (15) du boîtier de base (2),
- l'espace intérieur (15) est étanchéifié par rapport à une connexion fluidique (16) créée par la tubulure de liaison (4).

2. Ensemble selon la revendication 1, **caractérisé en ce que** la tubulure de liaison (4) fait partie du boîtier de base (2).

3. Ensemble selon la revendication 1 ou 2, **caractérisé en ce que** la tubulure de liaison (4) est réalisée sous forme de tubulure de raccordement d'un raccord.

4. Ensemble selon la revendication 3, **caractérisé en ce que** la tubulure de liaison (4) présente une contre-dépouille périphérique (23) qui s'insère dans une paroi interne du cathéter (3) et forme ainsi une denture avec cette paroi interne.

5. Ensemble selon la revendication 3, **caractérisé en ce que** le cathéter (3) peut être relié au boîtier de base (2) par une liaison à vis.

6. Ensemble selon la revendication 5, **caractérisé en ce que** la tubulure de liaison (4) présente un filetage intérieur ou extérieur par rapport à un filetage extérieur (28) ou intérieur (27) disposé à une extrémité de liaison (26) du cathéter (3).

7. Ensemble selon l'une quelconque des revendications 1 à 6, **caractérisé en ce que** l'unité de capteur de pression (6) est disposée en face d'un orifice (7) de la tubulure de liaison (4) dans le boîtier de base (2).

8. Ensemble selon l'une quelconque des revendications 1 à 7, **caractérisé en ce que** les composants électroniques (10) sont disposés dans au moins un espace (15) dans le boîtier de base (2) qui est étanche par rapport à la connexion fluidique (16) entre l'orifice de passage du cathéter (17) et l'unité de capteur de pression (6).

9. Ensemble selon l'une quelconque des revendications 1 à 8, **caractérisé en ce que** l'unité de capteur de pression (6) présente une surface sensible à la pression (13), réalisée sous forme de membrane, qui est en connexion fluidique directe (16) avec l'orifice de passage du cathéter (17).

10. Ensemble selon la revendication 9, **caractérisé en ce que** la surface sensible à la pression (13) porte un revêtement non organique.

11. Ensemble selon la revendication 9 ou 10, **caractérisé en ce que** la surface sensible à la pression (13) porte un revêtement de silicone.

12. Ensemble selon l'une quelconque des revendications précédentes, **caractérisé en ce que** le boîtier de base (2) est réalisé en céramique biocompatible ou en matière plastique biocompatible, notamment en polyuréthane, polyamide, fluoropolymère, silicone ou au moins un copolymère de ces matériaux.

13. Ensemble selon l'une quelconque des revendications précédentes, **caractérisé en ce que** le tuyau de cathéter est réalisé en matériau silicone, en silicones, en polyuréthanes, en polyamides, en polymères fluorés, en polyoléfines ou en copolymères de ces matériaux.

14. Ensemble selon l'une quelconque des revendications précédentes, **caractérisé en ce que** le joint annulaire (14) s'applique de manière périphérique contre une surface sensible à la pression (13) de l'unité de capteur de pression (6) et contre une paroi intérieure de la partie inférieure du boîtier (11) dans la zone de transition vers une tubulure de liaison (4).
